# FASCICULE DE BREVET EUROPEEN

(11) **EP 2 032 066 B1**
(45) Date de publication et mention de la délivrance du brevet: **24.08.2016**
(21) Numéro de dépôt: 07803940.1
(22) Date de dépôt: 26.06.2007
(51) Int. Cl.: A61N 2/00, A61N 5/01, A61B 34/35, A61B 90/11, A61B 90/50

(54) **INSTALLATION ROBOTISEE POUR LE POSITIONNEMENT ET LE DEPLACEMENT D'UN ORGANE OU INSTRUMENT ET APPAREIL DE TRAITEMENT COMPRENANT UNE TELLE INSTALLATION**
ROBOTERGESTEUERTE POSITIONIERUNGS- UND BEWEGUNGSEINRICHTUNG FÜR EIN ORGAN ODER INSTRUMENT UND BEHANDLUNGSEINRICHTUNG ENTHALTEND EINE SOLCHE EINRICHTUNG
ROBOTIZED INSTALLATION FOR THE POSITIONING AND MOVEMENT OF A COMPONENT OR INSTRUMENT, AND TREATMENT APPARATUS COMPRISING SUCH AN INSTALLATION

(30) Priorité: 26.06.2006 US 816343 P
(43) Date de publication de la demande: 11.03.2009
(73) Titulaire: Université de Strasbourg (Etablissement Public National à Caractère Scientifique, Culturel et Professionnel), 67000 Strasbourg (FR); Centre National de la Recherche Scientifique (CNRS) Etablissement Public à Caractère Scientifique et Technologique, 75016 Paris (FR); Institut National des Sciences Appliquees (Etablissement Public à Caractère Scientifique, Culturel et Professionnel), 67000 Strasbourg (FR)
(72) Inventeur: LEBOSSE, Cyrille, 2210 Luxemburg (LU); RENAUD, Pierre, 67300 Schiltigheim (FR); BAYLE, Bernard, 67000 Strasbourg (FR); DE MATHELIN, Michel, 67000 Strasbourg (FR); PICCIN, Olivier, 67170 Mittelhausen (FR); LAROCHE, Edouard, 67100 Strasbourg (FR)
(74) Mandataire: Nuss, Laurent
(86) Numéro de dépôt international: PCT/FR2007/051518
(87) Numéro de publication internationale: WO 2008/001003

(56) Documents cités:
- WO-A-03/098268
- DE-A1- 10 242 542
- DE-A1- 19 926 977
- US-A- 5 749 362
- US-A1- 2005 228 209
- US-B1- 6 428 206

## Description

La présente invention concerne le domaine des installations et dispositifs robotiques présentant une grande précision et une très bonne sécurité d'utilisation, permettant leur mise en oeuvre dans un contexte médical.

La présente invention a plus particulièrement pour objet une installation robotisée pour le positionnement et le déplacement d'une organe **sonde de stimulation magnétique transcrânienne,** un appareil de stimulation magnétique transcrânienne comprenant une telle installation robotisée et peut être utilisée dans le cadre d'un procédé de stimulation magnétique transcrânienne mettant en oeuvre l'appareil précité.

Dans de nombreuses procédures de traitement de patients ou d'imagerie médicale, il est nécessaire d'effectuer des positionnements et des déplacements précis et répétés d'organes ou d'instruments, ce, le cas échéant, sur des durées souvent conséquentes (plusieurs dizaines de minutes).

Ces manipulations sont fastidieuses et fatigantes pour l'opérateur, même lorsqu'elles sont assistées mécaniquement pour en faciliter l'exécution.

De plus, l'opérateur doit nécessairement être une personne spécialisée, c'est-à-dire généralement un chirurgien, un neurologue, un radiologue ou un spécialiste analogue en fonction des organes concernés et du type de traitement ou d'investigation à réaliser.

En outre, même un opérateur expérimenté, pouvant éventuellement exploiter un retour visuel fourni par un système de navigation, ne peut garantir un positionnement et un déplacement en accord optimal avec des données calculées préalablement, ni a fortiori la répétabilité à l'identique d'une procédure donnée, nécessitant plusieurs applications successives similaires pour être efficace.

Enfin, selon le type de traitement ou d'investigation à réaliser, l'opérateur manipulant manuellement l'organe ou l'instrument est exposé à un rayonnement nocif.

Ces différents facteurs expliquent la demande des praticiens pour la mise en oeuvre d'installations robotisés.

Plusieurs des problèmes exposés ci-dessus se posent notamment en relation avec la stimulation magnétique transcrânienne destinée à délivrer une stimulation électrique au cortex cérébral.

L'efficacité de ce procédé a été démontré dans le cas de la dépression et des études sont actuellement conduites pour d'autres pathologies telles que l'anxiété post-traumatique, les désordres obsessifs compulsifs, la schizophrénie et même pour certains types de désordres épileptiques.

Toutefois, on a observé une variabilité importante de l'efficacité selon le patient qui est principalement due à la difficulté de la manipulation de ce type de systèmes de stimulation tels qu'ils existent actuellement et qui conduit à une quasi impossibilité de reproduction à l'identique de la même procédure.

En effet, lors de la mise en oeuvre des procédures actuelles, dès que la zone cible du cortex a été définie par l'utilisation d'images d'IRM fonctionnelle, la sonde intégrant la bobine de stimulation magnétique doit être déplacée manuellement sur la tête du patient par le neurologue en vue de suivre une trajectoire précise dans l'espace.

Or, même lorsqu'un suivi visuel est fourni au neurologue par un système de navigation en vue de lui faciliter le positionnement de la bobine, il a été constaté que dans la pratique il était impossible d'obtenir un déplacement précis de la sonde.

De plus, un tel traitement manuel est extrêmement pénalisant en terme de coût de revient de la procédure, compte tenu de la nécessaire qualification de l'opérateur et de la durée importante de chaque séquence de traitement.

On connaît déjà différentes réalisations de dispositifs robotisés à usage médical, destinés à positionner et/ou à déplacer un organe ou un instrument par rapport à un patient.

Néanmoins, ces systèmes existants, souvent dérivés de dispositifs robotiques industriels, ne sont pas adaptés au déplacement d'un outil ou d'un instrument au niveau d'une surface d'un patient, ne font pas état d'une précision suffisante au niveau de leurs mouvements et/ou présentent un niveau de sécurité d'utilisation insuffisant pour une application médicale.

Par ailleurs, par le document "Psychiatry's schoking new tools" de Samuel K. Moore, IEEE Spectrum, Mars 2006, on connaît une représentation théorique d'un appareil de stimulation magnétique transcrânienne comprenant une structure articulée supportant la sonde d'application d'un champ magnétique pulsé.

Cette structure, pilotée automatiquement ou commandée à distance par un opérateur, comprend une potence télescopique à l'extrémité de laquelle est suspendue, par l'intermédiaire d'une liaison rotoïde, un sous-ensemble articulé portant la sonde.

Le sous-ensemble comprend lui-même un rail circulaire mobile dont l'une des extrémités est solidaire, par l'intermédiaire d'une liaison rotoïde, d'un rail glissière sur lequel est monté, de manière coulissante, la sonde.

Ainsi, la combinaison de ces différentes liaisons articulées ne fournit que cinq degrés de liberté et de ce fait seuls des points de la partie supérieure de la calotte crânienne peuvent être atteints par la sonde de cet appareil selon ce document.

De plus, il semble que cette structure ne permette pas de réaliser un balayage d'avant en arrière sur la tête du patient, et il faudrait compléter ladite structure par deux degrés de mobilité supplémentaires en translation dans un plan horizontal pour pouvoir balayer toute la surface supérieure du crâne.

Par ailleurs, aucun mécanisme de contrôle de l'orientation de la sonde de stimulation magnétique transcrânienne, autour d'un point de contact, n'est prévu.

Enfin, on note que la gestion de l'effort de contact, si elle est éventuellement prévue (le document IEE Spectrum n'en parle pas), serait difficile à mettre en oeuvre compte tenu de l'architecture de la structure. On note aussi qu'il n'est pas possible d'écarter la sonde selon un seul axe en cas d'incident, telle qu'une coupure de l'alimentation, sans risque d'interférer avec la tête du patient et que la multiplication des porte-à-faux est néfaste pour la rigidité de la structure et la précision des déplacements de la sonde.

La présente invention a pour objet de pallier au moins certains des inconvénients exposés ci-dessus et de proposer une solution robotisée par les différentes manipulations évoquées précédemment, relativement simple à commander et répondant aux critères requis de précision et de sécurité.

Par le document DE 10242542, on connaît un système motorisé de positionnement d'une sonde de stimulation magnétique transcrânienne (TMS) fournissant au plus quatre degrés de liberté, limitant ainsi considérablement les possibilités du système.

Par le document US-A-5 749 362, on connaît une installation d'imagerie robotisée permettant de réaliser une image d'un organe interne d'un patient, avec un manipulateur portant un instrument endoscopique.

Toutefois, comme déjà indiqué précédemment en relation avec le document IEEE Spectrum, l'installation proposée par ce document US fait état d'un nombre limité de degrés de liberté du manipulateur, ne permettant d'atteindre avec l'instrument porté qu'un nombre restreint de points du patient.

De plus, l'installation selon ce document US ne permet pas d'imposer une condition de tangence efficace de l'organe ou de l'instrument par rapport à la tête du patient, et n'est donc pas adaptée pour le positionnement d'une sonde TMS.

Afin de surmonter les limitations précitées et de pallier au moins certains des inconvénients évoqués précédemment, l'invention propose une installation robotisée selon le préambule de la revendication 1, présentant en outre les caractéristiques de la partie caractérisante de cette revendication.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
les figures 1 à 4 sont des vues respectivement en perspective, en élévation latérale, en élévation frontale et de dessus d'une installation robotisée selon l'invention mise en oeuvre en relation avec une sonde de stimulation magnétique transcrânienne,
les figures 5A et 5B sont des vues en perspective, selon deux angles légèrement différents, de l'installation robotisée des figures 1 à 4, l'enveloppe d'habillage ayant été enlevée,
la figure 6A est un schéma cinématique équivalent d'un mode de réalisation, à quatre degrés de liberté, du dispositif robotique faisant partie de l'installation robotisée représentée aux figures 1 à 5 ou de deux sous-ensembles constitutifs de ce dispositif, **ne faisant pas partie de l'invention,**
la figure 6B est un schéma cinématique équivalent d'un mode de réalisation à sept degrés de liberté du dispositif robotique faisant partie de l'installation robotisée représentée sur les figures 1 à 5, **en accord avec l'invention,**
les figures 7A et 7B sont des vues en perspective de l'installation robotisée des figures 1 à 5 illustrant les positions extrêmes, respectivement supérieure et inférieure, de la première articulation rotatoire du premier sous-ensemble du dispositif robotique et montrant le mouvement au niveau de cette articulation,
les figures 8A, 8B, 8C et 8D sont des vues similaires à celles des figures 7A et 7B illustrant quatre positions décalées successivement de 90° de la deuxième articulation rotatoire du premier sous-ensemble du dispositif robotique et montrant le mouvement au niveau de cette articulation,
les figures 9A et 9B sont des vues similaires à celle de la figure 8D illustrant les positions extrêmes (déployée et repliée) de la troisième articulation rotatoire du premier sous-ensemble du dispositif robotique et montrant le mouvement au niveau de cette articulation,
la figure 10 est une représentation maillée et en relief de la tête d'un patient illustrant la zone accessible pour l'organe ou instrument porté par le dispositif robotique sans qu'il y ait interférence avec le patient,
les figures 11A et 11B sont des vues partielles en perspective selon deux angles différents, représentant le deuxième et le troisième sous-ensembles du dispositif robotique faisant partie de l'installation robotisée des figures 1 à 5 et illustrant les deux positions extrêmes en translation du deuxième sous-ensemble du dispositif robotique et montrant son axe de déplacement,
la figure 12 est une représentation partielle en élévation latérale de l'objet des figures 11A et 11B illustrant les mouvements rotatoires possibles au niveau des première, deuxième et troisième articulations faisant partie du troisième sous-ensemble du dispositif robotique,
la figure 13 est une vue partielle en perspective du troisième sous-ensemble du dispositif robotique faisant partie de l'installation robotisée, certains des éléments constitutifs présentant une structure et un arrangement légèrement différent du troisième sous-ensemble représenté aux figures 11A, 11B et 12,
la figure 14 est une vue partielle du dessus de l'installation robotisée telle que représentée aux figures 5A et 5B montrant partiellement certains éléments des articulations du premier sous-ensemble du dispositif robotique,
la figure 15 est une vue partielle en élévation latérale de l'installation robotisée telle que représentée à la figure 5 montrant plus particulièrement les deuxième et troisième articulations rotatoires du premier sous-ensemble du dispositif robotique, ainsi que l'articulation coulissante formant le deuxième sous-ensemble du dispositif robotique,
la figure 16 est une vue partielle de détail et en perspective de l'installation robotisée telle que représentée à la figure 5 montrant plus particulièrement le siège formant dispositif de support et de maintien du patient, ainsi que les dispositifs de réglages vertical et horizontal qui lui sont associés,
les figures 17 et 18 sont des vues partielles de détail et en perspective de l'installation robotisée telle que représentée à la figure 5 montrant plus particulièrement les éléments constitutifs du dispositif de sécurité associé à la première articulation rotatoire du premier sous-ensemble du dispositif robotique et réalisé sous la forme d'un mécanisme de rappel à tension constante,
les figures 19 et 20 sont des vues partielles de détail et en perspective de l'installation robotisée telle que représentée à la figure 5 montrant plus particulièrement les éléments constitutifs des première et deuxième articulations rotatoires du premier sous-ensemble du dispositif robotique,
les figures 21A, 21B, 21C, 21D et 21E sont des vues partielles de détail et en perspective de l'installation robotisée telle que représentée à la figure 5 montrant plus particulièrement les éléments du circuit de circulation d'air pour le refroidissement de la sonde, ainsi que le câble d'alimentation de la sonde, au niveau de la deuxième articulation rotatoire du premier sous-ensemble du dispositif robotique, et,
la figure 22 est une vue en perspective d'une sonde de stimulation magnétique transcrânienne faisant partie d'un appareil de traitement comprenant l'installation robotisée représentée aux figures 1 à 5.

Ces dernières montrent une installation robotisée 1 pour le positionnement et le déplacement guidés et contrôlés d'un organe ou instrument 2 de traitement, **du type** sonde de stimulation magnétique transcrânienne ou analogue, au niveau ou autour de la tête 3 d'un patient 3'.

Cette installation 1 comprend essentiellement une structure support (bâti) 4, revêtue d'une enveloppe d'habillage et de protection 4' et sur laquelle sont montés les éléments constitutifs 7 à 31 d'un dispositif robotique 5 formant une chaîne cinématique série et portant à son extrémité libre et contrôlée en position **la sonde** 2 précitée. De plus, un dispositif 6 réglable de support et de maintien du patient 3', sensiblement en position assise, fait partie de ou est associé à ladite structure support 4.

Conformément à l'invention, ledit dispositif robotique 5 est constitué de trois 7, 7', 7", sous-ensembles cinématiques mutuellement associés en série, comprenant, d'une part, un premier sous-ensemble 7 sous la forme d'un mécanisme à articulations rotatoires 8, 8', 8", solidaire de la structure support 4 par une première articulation 8 et correspondant à un arrangement cinématique sphérique de type série à trois degrés de liberté, les éléments d'articulation 8, 8', 8" étant tous situés en dehors du volume 9 susceptible de recevoir le patient 3' et leurs axes de rotation axe 1, axe 2 et axe 3 étant concourants en un point focal PF correspondant sensiblement au centre hypothétique de la tête 3 du patient 3' en position d'intervention d'autre part, un deuxième sous-ensemble 7' sous la forme d'un mécanisme à translation linéaire selon un axe (axe 4) passant par le point focal PF précité et solidaire de la partie mobile 10 de la troisième articulation 8" en série du premier sous-ensemble 7, et enfin un troisième sous ensemble 7" sous la forme d'un second mécanisme à articulations rotatoires, solidaire de la partie mobile 12 du second sous-ensemble 7' et correspondant également à un arrangement cinématique sphérique de type série à trois degrés de liberté, les éléments d'articulation 11, 11', 11" de ce troisième sous-ensemble 7" présentant des axes de rotation axe 5, axe 6, axe 7 concourants.

La décomposition du dispositif robotique 5 en trois sous-ensembles 7, 7', 7" indépendants et reliés entre eux en série facilite la commande en position et en déplacement, augmente la précision du mouvement résultant et permet de sécuriser individuellement chacun des éléments d'articulation qui le constituent.

En particulier l'association d'un premier sous-ensemble 7 à mouvement sphérique, formé de trois liaisons rotatoires ou rotoïdes en série (8/axe 1, 8'/axe 2, 8"/axe 3) et non interférant avec le volume 9 recevant le patient 3', avec un deuxième sous-ensemble 7' à mouvement en translation selon un axe (axe 4) radial par rapport à la sphère ou la partie de sphère dont les points peuvent être atteints au moyen du premier sous-ensemble 7, permet une gestion optimisée de la sécurité du dispositif robotique 5, éventuellement par le seul contrôle sécurisé de ce deuxième sous-ensemble 7'.

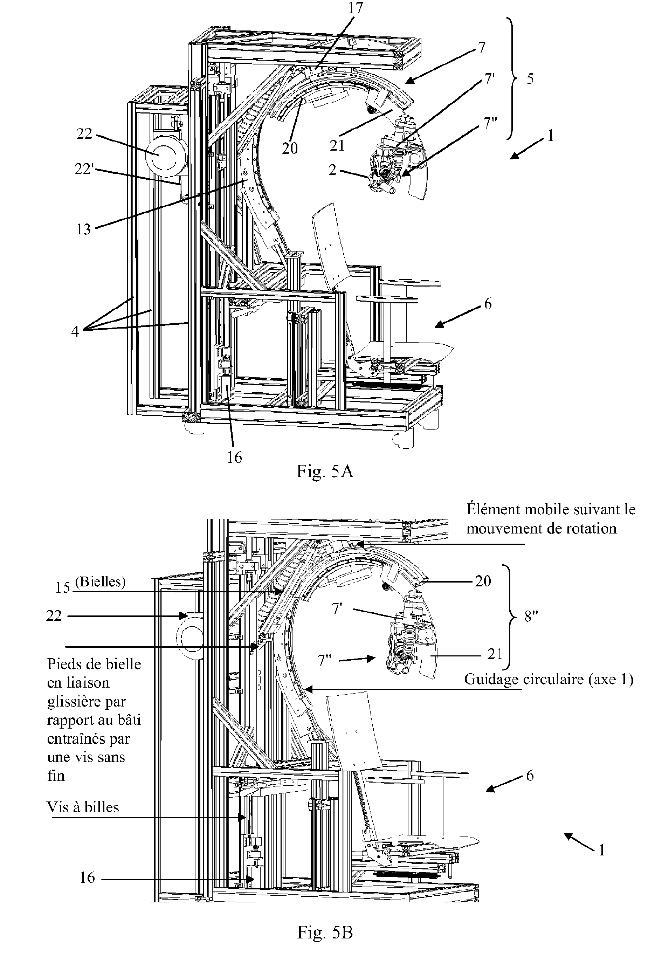

En accord avec une première caractéristique de l'invention, ressortant notamment des figures 5, 14, 17, 19 et 20, la première articulation rotatoire 8 du premier sous-ensemble 7 consiste en une liaison à glissière circulaire, comprenant un rail de guidage 13, préférentiellement un rail double, en forme d'arc de cercle, sensiblement semi-circulaire, solidaire de la structure support 4, et un chariot mobile 14 apte à circuler sur ledit rail 13, et dont le déplacement est commandé par une tringlerie de transmission 15 reliée à un actionneur 16, le mouvement circulaire s'effectuant dans le plan médian du patient 3 en position d'intervention et ledit rail de guidage 13 présentant un positionnement et une extension tels qu'il s'étend autour de la tête 3 dudit patient 3' sensiblement de la base arrière du crâne jusqu'au front.

Le rail 13 est fixé rigidement en plusieurs endroits à la structure support 4, au niveau d'une partie supérieure en forme de potence surplombant le dispositif de support et de maintien de patient, sous forme de siège réglable 6.

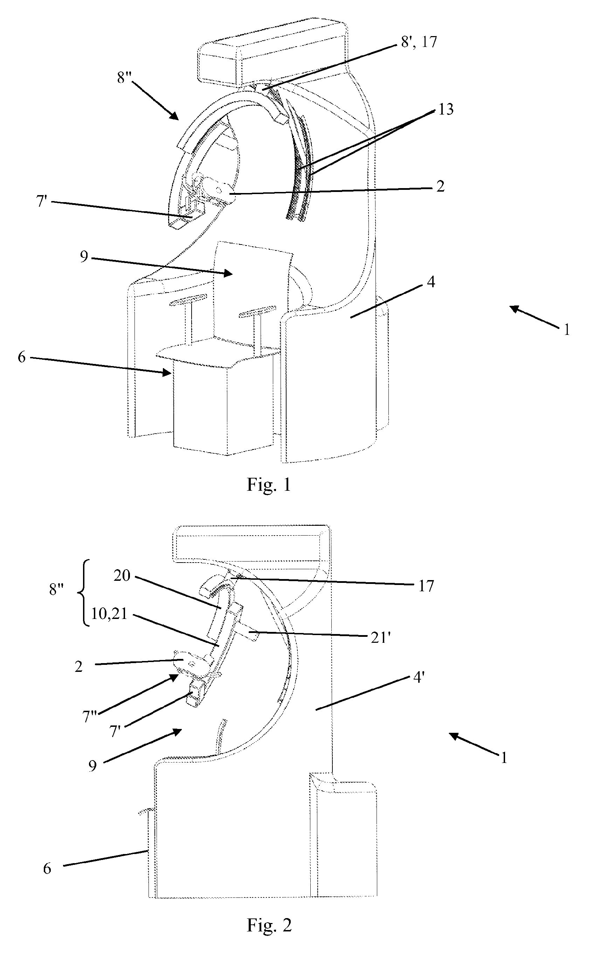

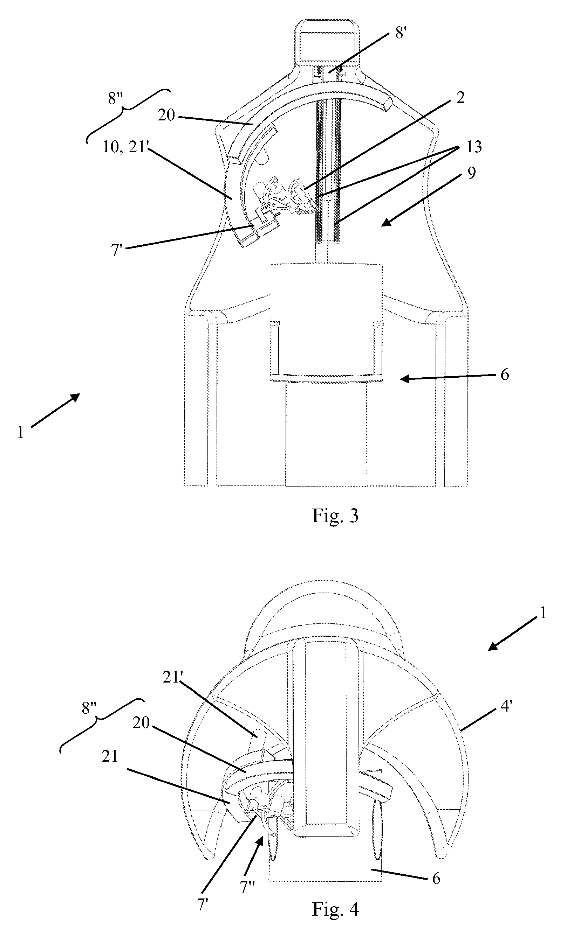

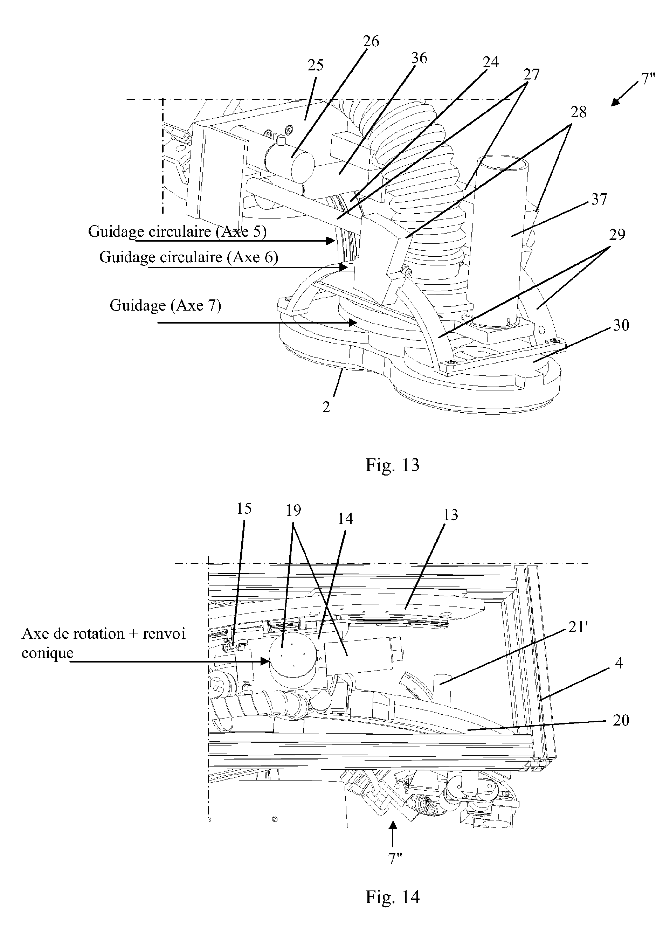

Le rail 13 peut présenter soit une structure double (figures 1 à 3, 7 à 9, 17, 19 et 20), soit une structure à rail unique (figures 5A, 5B et 14). Dans le premier cas, le chariot 14 circule, au niveau de gorges adaptées, sur les deux rails identique et parallèles entre eux par l'intermédiaire de patins à roulement ou de portions de glissières situés de part et d'autre dudit chariot 14, et dans le second cas précité, le chariot 14 circule sur le rail unique par l'intermédiaire d'au moins deux patins à roulement ou à glissières situés l'un à la suite de l'autre, sur un seul côté.

La tringlerie de transmission 15, réalisant le déplacement du chariot 14 sur le rail 13, peut comprendre comme cela ressort des figures 5A et 5B une paire de bielles reliées au chariot 14 par une extrémité et à un second chariot coulissant verticalement dans la structure support 14 par l'autre extrémité. Ce second chariot peut être entraîné en translation par le moteur 16 par l'intermédiaire d'une vis à billes et d'une vis mère sans fin engageant ledit second chariot au niveau d'un écrou fils (figure 5B).

L'utilisation d'un premier sous-ensemble 7 de type mécanisme articulé sphérique série à trois degrés de liberté est particulièrement adaptée à un volume de travail de forme sphérique, tel que l'espace entourant la tête d'un patient, le positionnement de **la sonde** 2 étant réalisé au niveau d'une sphère centrée sur la tête du patient.

Le sous-ensemble 7 consiste selon l'invention en un arrangement particulier de guides circulaires (deux sous-assemblages concentriques à guidage circulaire reliés entre eux par un axe de rotation), évitant ainsi toute interférence avec le patient et optimisant la rigidité du dispositif pour faciliter la manipulation précise de **la sonde** 2.

Les mécanismes de sécurité associés aux trois articulations rotatoires 8, 8', et 8" garantissent la sécurité du patient en cas d'incident.

Selon une autre caractéristique de l'invention et comme le montrent les figures 14, 17, 19, 20, 21A et 21B, la seconde articulation rotatoire 8' du premier sous-ensemble 7 consiste en une articulation axiale avec un arbre 17 monté à rotation dans un palier 18 ménagé dans le chariot mobile 14 faisant partie de la première articulation rotatoire 8, le déplacement en rotation dudit arbre étant commandé par un actionneur 19, par exemple une unité moteur - réducteur, porté par ledit chariot 14.

Conformément à une autre caractéristique de l'invention, ressortant notamment des figures 5, 9, 15 et 19, la troisième articulation rotatoire 8" du premier sous-ensemble 7 consiste en une liaison à glissière circulaire, préférentiellement sous la forme de deux rails 20, 21 coopérant de la manière coulissante et réalisé chacun en forme d'arc de cercle, lesdits rails 20 et 21 pouvant être déplacés relativement l'un par rapport à l'autre entre une disposition repliée dans laquelle ils sont sensiblement superposés ou chevauchant sur toute leur longueur, et une disposition déployée, dans laquelle ils ne sont plus superposés que sur une faible partie.

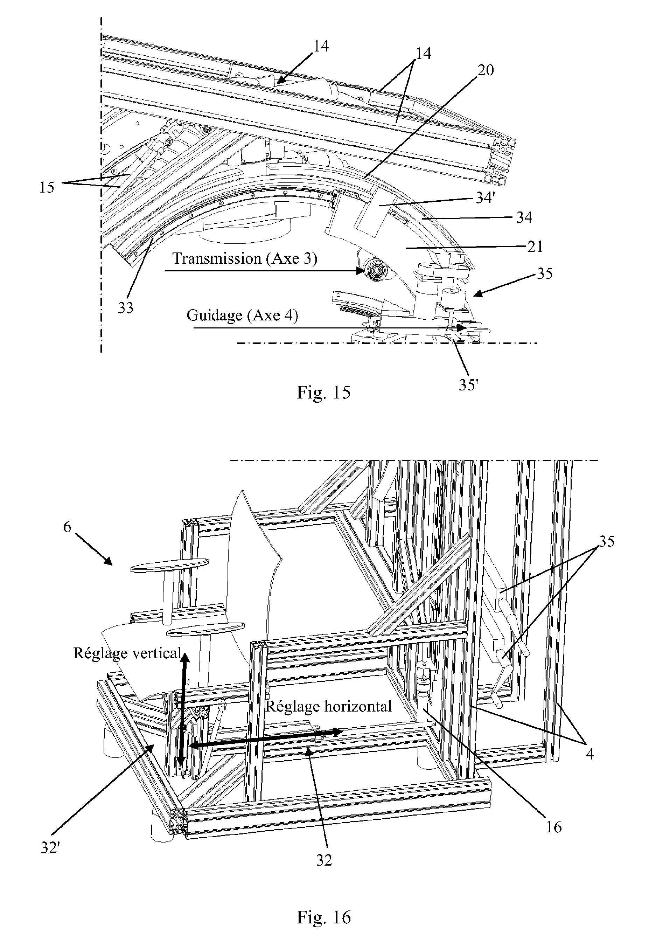

Comme le montre à titre d'exemple de réalisation pratique la figure 15, le rail fixe 20 peut être équipé d'un profilé de roulement 33 s'étendant sur toute sa longueur et le rail 21 être pourvu de plusieurs trains de roulements à billes ou à aiguilles répartis sur sa longueur.

En outre, le rail fixe 20 peut être muni d'une règle optique 34 et le rail mobile 21 d'un capteur optique correspondant 34'.

Le déplacement du rail mobile 21 par rapport au rail fixe 20 pourra être réalisé, par exemple, par l'intermédiaire d'un système à crémaillère ou à galet d'entraînement, entraîné au moyen d'une transmission adaptée par un moteur électrique porté par le rail mobile 21.

Comme le montrent également les figures précitées, le rail fixe 20 est assemblé rigidement avec l'arbre 17 de la seconde articulation rotatoire 8', les deux rails 20 et 21 pouvant être bloqués en position l'un par rapport à l'autre constructivement, par liaison mécanique ou par blocage de l'actionneur assurant leur mouvement relatif mutuel.

En cas de blocage de cette troisième articulation rotatoire 8", le premier sous-ensemble 7 constitue un mécanisme articulé sphérique à deux degrés de liberté, qui peut être suffisant pour certaines applications nécessitant une mobilité réduite pour **la sonde** 2.

Le blocage de cette articulation 8" peut être définitif ou temporaire, et dans ce dernier cas il pourra être obtenu consécutivement à une programmation logicielle correspondante de l'unité de commande du moteur électrique assurant la mobilité du rail 21 relativement au rail 20.

L'arbre 17 reliant le rail 20 de manière rotatoire au chariot 14 présente préférentiellement une longueur relativement faible, de manière à réduire le porte-à-faux entre les deux éléments reliés et assurer une bonne rigidité à l'assemblage articulé réalisé.

Afin d'éviter une possible chute du dispositif robotique 5 dans le volume 9 et d'obtenir un dégagement automatique vers le haut dudit dispositif, il peut être avantageusement prévu que le chariot mobile 14 de la première articulation rotatoire 8 soit de manière permanente sollicité en position extrême supérieure par un mécanisme de rappel à tension constante 22, par exemple à câble 22' (figures 5, 17 et 18).

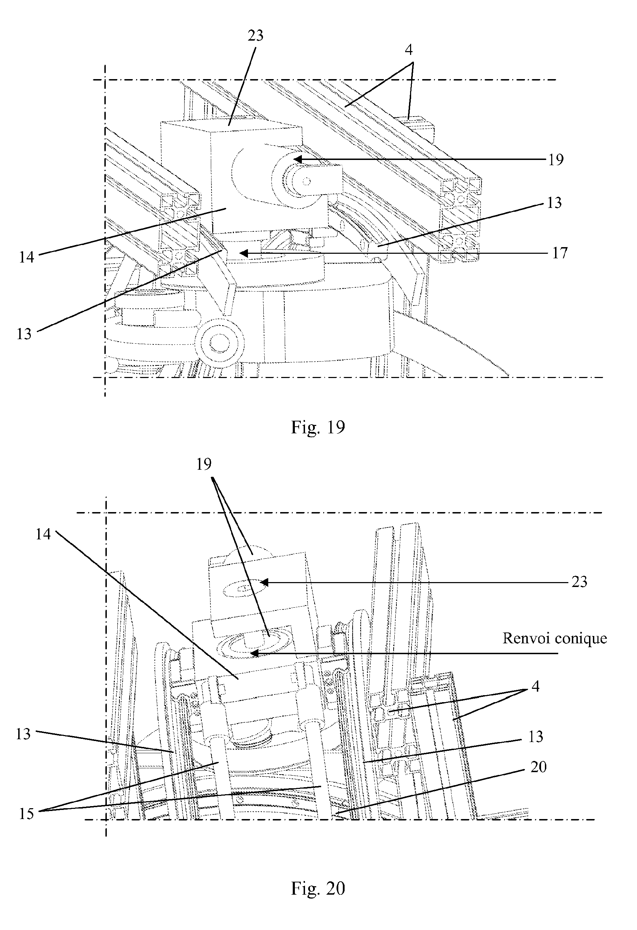

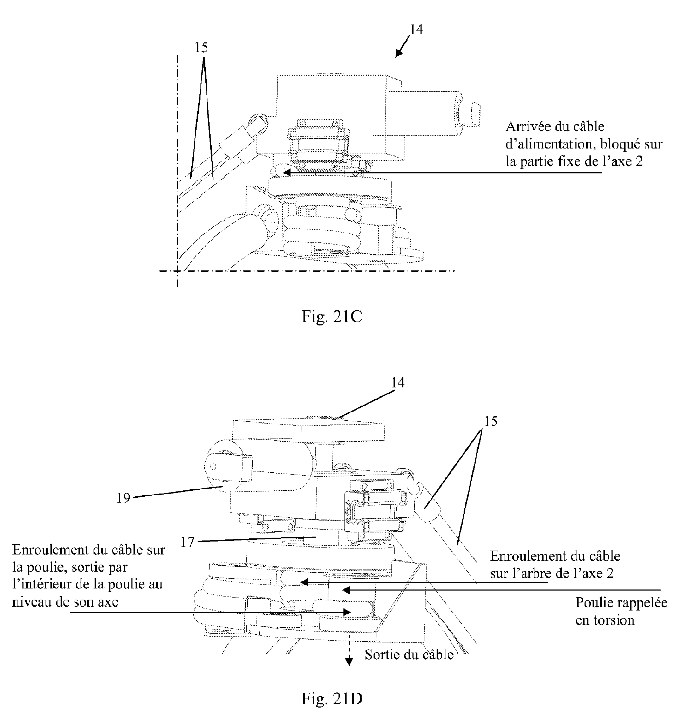

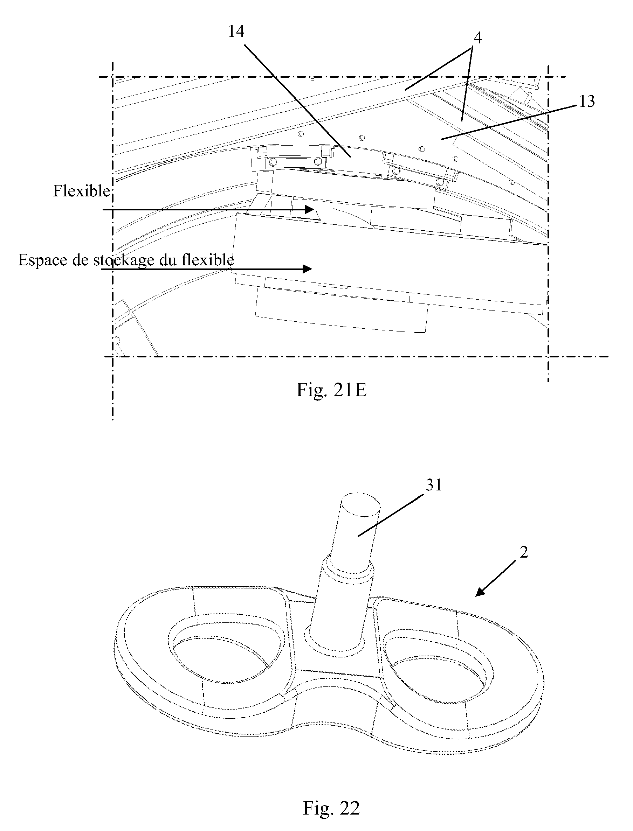

De plus, en vue d'éviter tout mouvement non contrôlé des éléments fixés à l'arbre 17, pouvant éventuellement interférer avec le patient 3', un dispositif 23 de blocage en rotation de l'arbre 17 est associé à l'actionneur 19, par exemple sous la forme d'un frein actif en l'absence d'alimentation électrique au niveau de ce dernier, par exemple du type électromagnétique, ledit dispositif 23 pouvant être débloqué par un opérateur par l'intermédiaire d'une commande manuelle d'alimentation en courant, afin d'autoriser une libre rotation de l'arbre 17 autour de son axe de rotation axe 2 (figures 20 et 21D).

A titre de sécurité supplémentaire au niveau du premier sous-ensemble 7, il peut également être prévu que l'actionneur 21' de la troisième articulation rotatoire 8" du premier sous-ensemble 7 réalise un blocage en position mutuel des deux rails 20 et 21 en l'absence d'alimentation électrique.

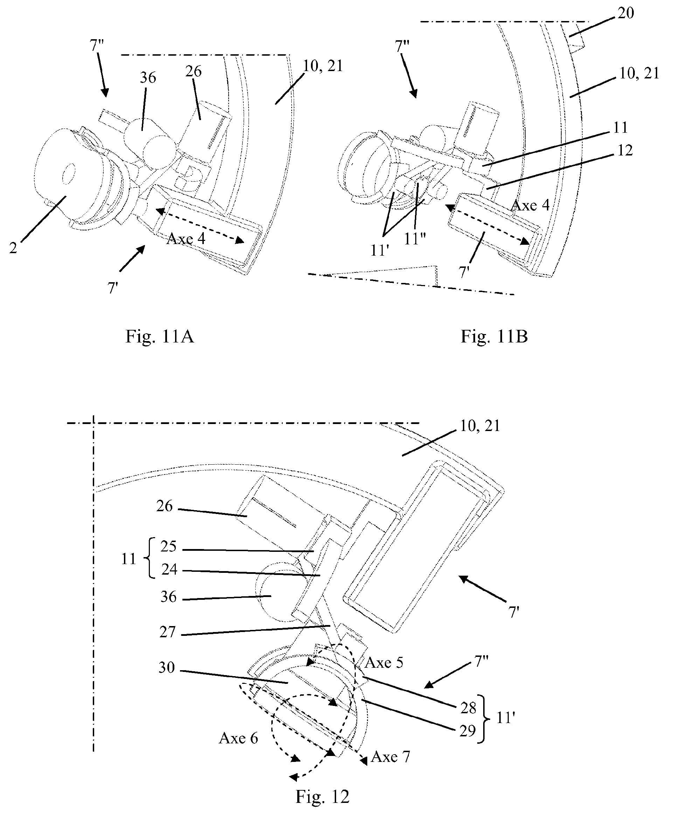

Conformément à une caractéristique de l'invention ressortant notamment des figures 11 et 15, permettant de contrôler la pression d'application de **la sonde** 2 contre la tête 3 du patient 3', un capteur d'effort est associé au deuxième sous-ensemble 7', permettant de réaliser un asservissement en effort dudit deuxième sous-ensemble 7' dans la direction de translation (axe 4) sensiblement perpendiculaire à la surface de la tête 3 du patient 3' en position d'intervention, ce sous-ensemble 7' comprenant en outre un mécanisme de rappel, par exemple de type mécanique, sollicitant **la sonde** 2 à distance de la surface de la tête 3 du patient 3'.

Le capteur d'effort peut être soit monté dans le sous-ensemble 7', soit être intégré directement dans **la sonde** 2.

En particulier dans le cas d'une sonde de stimulation magnétique transcrânienne ou analogue (**sonde** 2 destiné à venir en application contre la tête d'un patient), ledit capteur d'effort peut être intégré dans **la sonde** 2 porté par le dispositif robotique 5, ledit capteur d'effort faisant partie d'un asservissement en effort du deuxième sous-ensemble 7' dudit dispositif 5.

Le capteur d'effort pourra alors se présenter sous la forme d'un capteur mince en forme de portion de feuille tel que, par exemple, les capteurs connus sous la désignation FlexiForce (nom déposé) de la société Tekscan ou sous la désignation FSR (nom déposé) de la société Interlink Electronics. Ce capteur sera insensible au rayonnement éventuellement émis par **la sonde** 2.

La figure 15 montre (partiellement en coupe) l'ensemble 35 moteur / codeur assurant l'actionnement contrôlé de la liaison glissière formant le deuxième sous-ensemble 7', ainsi que le ressort de rappel formant le mécanisme de sécurité de cette liaison.

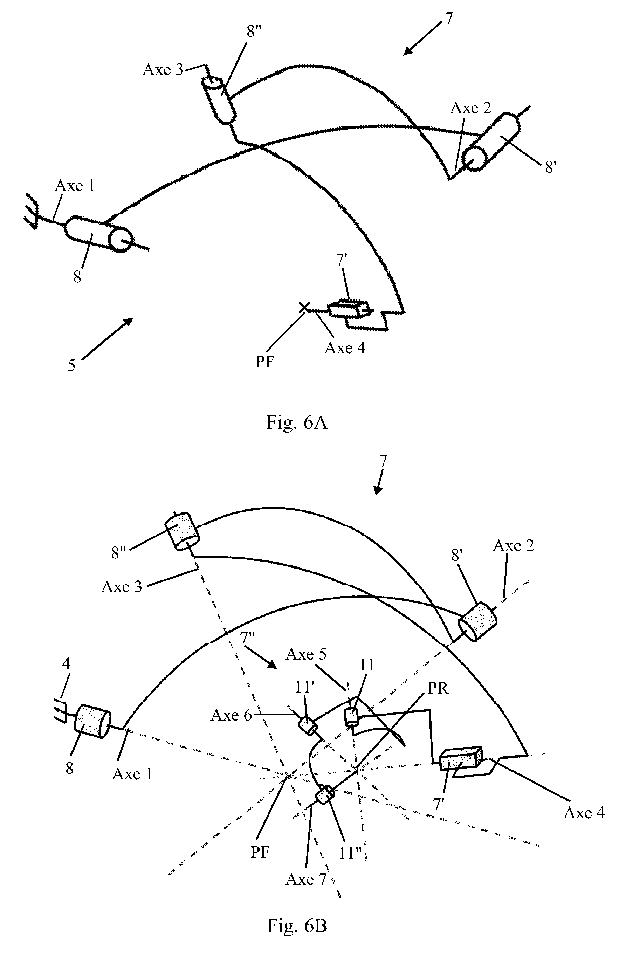

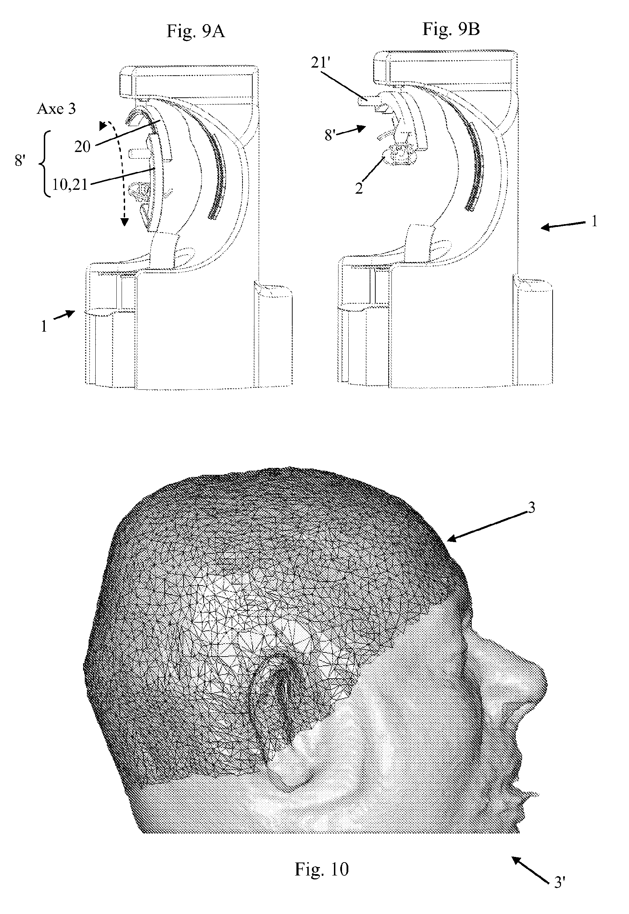

Selon une première variante de réalisation **ne faisant pas partie** de l'invention, représentée de manière schématique et cinématique sur la figure 6**A**, l'installation robotisée 1 comprend un dispositif robotique 5 à quatre degrés de liberté, intégrant les premier et deuxième sous-ensembles 7 et 7', un tel dispositif permettant d'atteindre avec **la sonde** 2 l'ensemble des points représentés sur la figure 10.

En accord avec l'invention ressortant des figures 1 à 5 et 7 à 13 et représenté schématiquement et cinématiquement sur la figure 6B, l'installation robotisée 1 comprend un dispositif robotique 5 redondant à sept degrés de liberté, intégrant les premier, deuxième et troisième sous-ensembles 7, 7' et 7", le troisième sous-ensemble 7" consistant en un mécanisme à trois articulations rotatoires en série 11, 11', 11" formant un poignet sphérique.

Un tel dispositif robotique présente non seulement les mêmes propriétés que la première variante précitée (qu'elle intègre), mais permet également de réaliser une orientation locale de **la sonde** 2 par rapport à un point de référence donné PR, par exemple le point de contact ou point central de la surface de contact entre **la sonde** 2 et la tête 3 du patient 3' (éventuellement confondu avec le point central de **la sonde** 2).

Cette propriété supplémentaire est notamment nécessaire dans le cadre de la stimulation magnétique transcrânienne pour assurer la condition de tangence entre la sonde 2 et la tête 3 durant la procédure de traitement nécessitant un mouvement de balayage contrôlé de ladite sonde.

En accord avec une construction pratique de l'invention, représentée plus particulièrement aux figures 11 à 13 des dessins annexés, le troisième sous-ensemble 7" formant le poignet sphérique est essentiellement constitué par une première articulation 11 formée d'un rail 24 en arc de cercle solidaire de la partie mobile 12 du deuxième sous-ensemble 7' et sur lequel circule un chariot 25 dont le déplacement est commandé par un actionneur 26, par une seconde articulation 11' formée de deux bras 27 montés rigidement sur le chariot mobile 25 par l'une de leur extrémités et portant à leurs extrémités opposées chacun un chariot fixe 28 coopérant chacun (coulissement curviligne)avec une portion de rail mobile 29 en forme d'arc de cercle et parallèles entre elles, et par une troisième articulation 11" sous la forme d'une platine à palier 30, solidaire des rails mobiles 29 et apte à recevoir avec faculté de rotation (autour de axe 7) un moyeu 31 solidaire de **la sonde** 2.

L'actionneur 36 (par exemple un moteur électrique) assurant le déplacement des rails mobiles 29 dans les chariots 28 peut, par exemple, être porté par les bras 27, la transmission du mouvement étant assurée à travers ou autour de ces dernières (cabestans).

De même, l'actionneur 37 (moteur électrique) assurant la rotation de **la sonde** 2 autour de son moyeu 31 (axe 7) pourra être directement porté par la platine à palier 30 recevant **la sonde** 2.

Les plages angulaires maximales pour les articulations 11, 11' et 11" formant le troisième sous-ensemble 7", ainsi que pour les articulations 8, 8' et 8", sont déterminées lors de la conception du dispositif robotique 5 en évaluant (sur la base d'une reconstitution tridimensionnelle de la tête du patient à partir d'images IRM par exemple) les angles nécessaires pour assurer la tangence de la face frontale de la sonde 2 au niveau des différentes régions de la tête à atteindre.

De plus, de possibles décalages entre le centre de la tête du patient et le point focal PF sont également à prendre en compte dans la réalisation du sous-ensemble 7".

Une description complémentaire de l'invention, en relation directe avec les dessins annexés, est présentée ci-après, plus précisément mais non limitativement, dans le cadre d'une application à la stimulation magnétique transcrânienne.

Comme le montrent les figures 1 à 5 et 7 à 9, l'installation robotisée 1 comprend essentiellement une structure support rigide 4 (par exemple sous la forme d'un assemblage de profilés métalliques amagnétiques), un carter (enveloppe ou habillage 4'), un siège réglable 6, et un dispositif robotique 5 présentant sept degrés de liberté. Le dispositif robotique 5 permet le positionnement d'une **sonde** 2 par déplacement autour d'un point fixe PF dans l'espace. Il permet notamment le positionnement d'une sonde 2 d'imagerie ou de stimulation autour de la tête 3 d'un patient 3'. Tout autre dispositif devant être maintenu en contact de manière sûre avec la tête peut également être envisagé.

Dans le cas des figures précitées, le dispositif robotique 5 est représenté avec un effecteur (sonde 2) permettant la réalisation d'un traitement de stimulation magnétique transcrânienne (SMT).

Comme cela ressort des figures 1 à 9, 10 à 15 et 17 à 20, le dispositif robotique 5 est composé de trois sous-ensembles 7, 7' et 7" montés en série: une structure principale 7 à trois degrés de liberté, une liaison glissière 7' actionnée et un poignet sphérique 7" à trois degrés de liberté.

La structure principale 7 permet le placement et le déplacement du centre de la sonde de stimulation magnétique transcrânienne 2 sur une sphère centrée sur le patient 3'. Il s'agit sur un plan cinématique d'une structure de robot série sphérique (figure 6).

L'utilisation, pour la réalisation des première et troisième articulations rotatoires 8 et 8", de guidages circulaires, rend possible l'obtention de mouvements sans interférences avec le patient 3', et en assurant la rigidité nécessaire du dispositif 5.

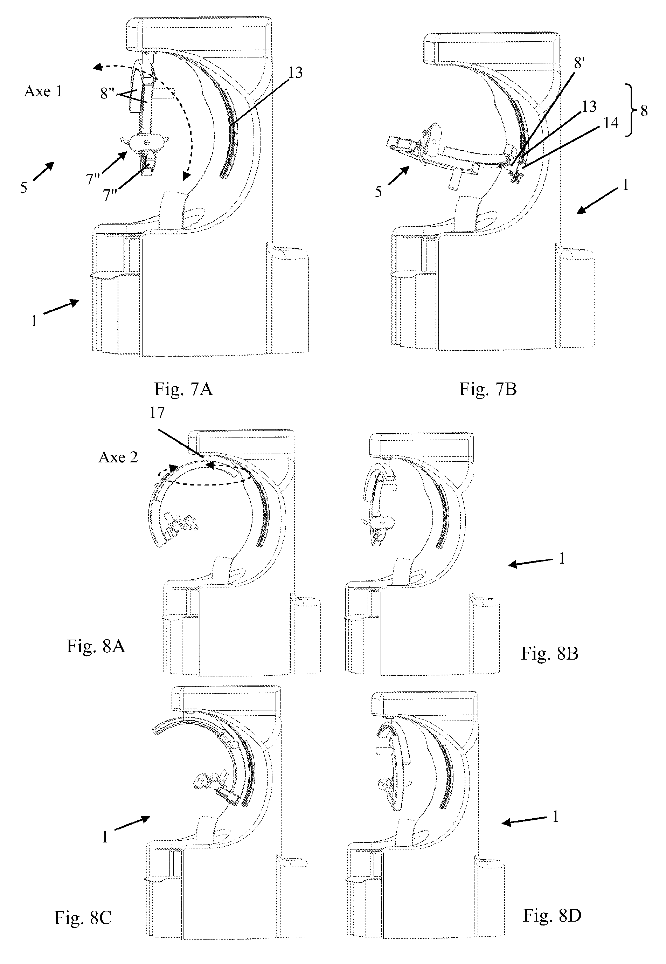

Les déplacements maximums pouvant être obtenus au niveau de chacune des articulations 8, 8' et 8" sont indiqués sur les figures 7 à 9. Ces déplacements permettent d'atteindre la surface maillée représentée sur la figure 10: cette surface correspond à l'ensemble de la calotte crânienne, délimitée par le cerveau (partie antérieure et supérieure du crâne, front, tempes, oreilles). La disposition des liaisons (articulations 8, 8' et 8") et l'amplitude possible des déplacements au niveau de ces liaisons rotatoires ou rotoïdes permettent d'assurer un déplacement sur cette zone avec une précision compatible avec le traitement de SMT, en obtenant en particulier un indice d'isotropie pour la structure qui est supérieur à 0,8.

Comme le montrent les figures 6, 11, 12 et 15, la liaison glissière actionnée (deuxième sous-ensemble 7') permet le déplacement de la sonde 2 en contact avec le patient 3'. La direction d'actionnement (axe 4), proche de la normale à la surface de la tête 3 du patient, permet de gérer simplement l'effort de contact sonde/patient: l'asservissement en effort est obtenu par asservissement de cet unique axe (axe 4). La liaison glissière 7' est rappelée en position par un dispositif mécanique (par exemple du type ressort précontraint). En cas de coupure de courant, la sonde 2 tend à s'éloigner du patient assurant la sécurité de ce dernier.

Le poignet sphérique (sous-ensemble 7" / figures 11, 12 et 13) permet d'imposer la condition de tangence du plan de la sonde 2 à la tête 3 du patient, nécessitée par le traitement. La rotation propre de la sonde (axe 7) permet d'orienter le champ magnétique, et d'exciter au mieux les sillons corticaux.

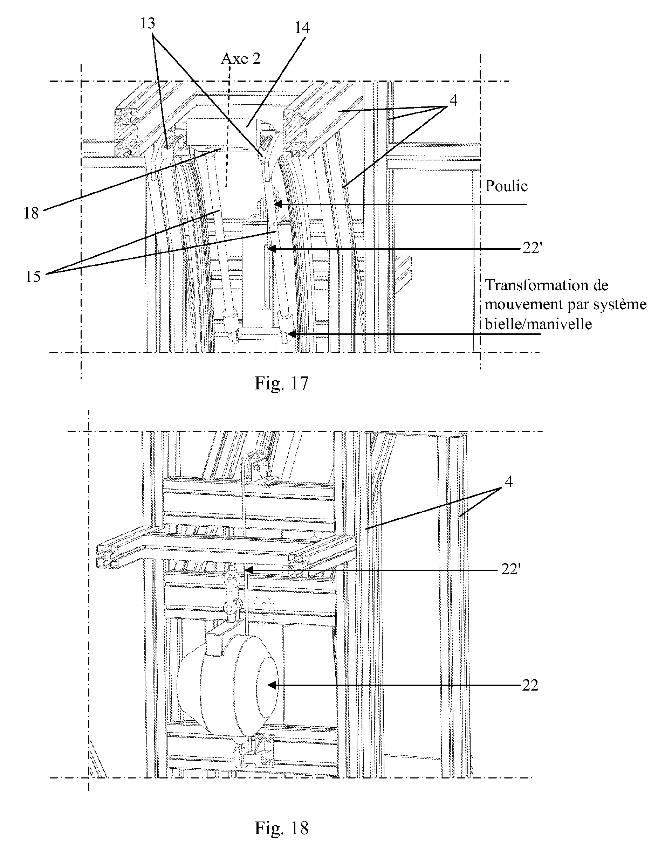

Le comportement du dispositif robotique 5 est sûr en cas de coupure volontaire ou involontaire de la puissance (arrêt d'urgence, fin d'utilisation, coupure de courant): l'articulation rotatoire 8 autour de l'axe 1 de mouvement en rotation revient alors en position verticale haute (figure 7A) par un système de rappel à tension constante (figures 17 et 18), évitant toute chute du dispositif sur le patient.

De même, le dispositif de rappel associé à la liaison glissière éloignera automatiquement la sonde de la tête 3 du patient en cas d'incident similaire.

Comme le montrent plus particulièrement les figures 14, 15, 19 et 20, l'articulation 8' fournissant un déplacement en rotation autour de l'axe de l'arbre 17 (axe 2) est pilotée à l'aide d'un moteur électrique embrayant sur un axe perpendiculaire (arbre 17). Cet axe permet l'obtention de la rotation désirée par l'intermédiaire d'un réducteur, par exemple, un pignon conique engrenant avec une roue dentée solidaire de l'arbre 17. Ce dernier est également connecté à un dispositif de freinage 23 par manque de courant. En cas de suppression de ce dernier, le freinage et le blocage de la rotation est assuré, évitant tout mouvement non contrôlé autour de l'articulation 8', et des interférences éventuelles avec le patient.

La sortie du volume 9 doit cependant être possible même en cas de défaillance de l'installation robotisée 1. Si à cet instant la sonde 2 est par exemple située face au patient 3', le gênant dans son mouvement de sortie, un opérateur peut intervenir manuellement pour envoyer ponctuellement un courant dans le frein 23 et orienter manuellement le dispositif robotique 5 (dans l'une des positions des figures 8A et 8D).

La figure 22 représente une sonde SMT de forme classique au niveau de la constitution générale et comportant un moyen arrière 31 autorisant son montage à rotation dans la platine à palier 30 du poignet sphérique formant le troisième sous-ensemble 7".

La sonde comprend comme élément actif un enroulement de cuivre en forme de 8, permettant la création d'un champ magnétique intense. Le bon fonctionnement de la sonde 2 requiert son refroidissement, par exemple par circulation d'air.

En effet, la création du champ magnétique au niveau de la sonde 2 de SMT provoque l'échauffement du conducteur en cuivre qui la compose. Pour assurer un fonctionnement correct, un refroidissement doit donc être assuré.

Par ailleurs, les mouvements du patient 3' sont détectés à l'aide d'un localisateur externe, permettant de déterminer la position et l'orientation de la tête du patient. Afin d'assurer le bon fonctionnement de ce localisateur, il est nécessaire de minimiser la présence de câbles et de flexibles autour du dispositif robotique 5. La présence de câbles pourrait de plus gêner le patient ou l'opérateur au cours de l'utilisation de l'appareil.

En conséquence, le dispositif robotique 5 est conçu de manière à intégrer un flexible permettant le refroidissement de la sonde 2 de SMT par aspiration d'air depuis un aspirateur ou dispositif à vide analogue intégré à l'installation 1, ainsi que le câble d'alimentation permettant le fonctionnement de la sonde.

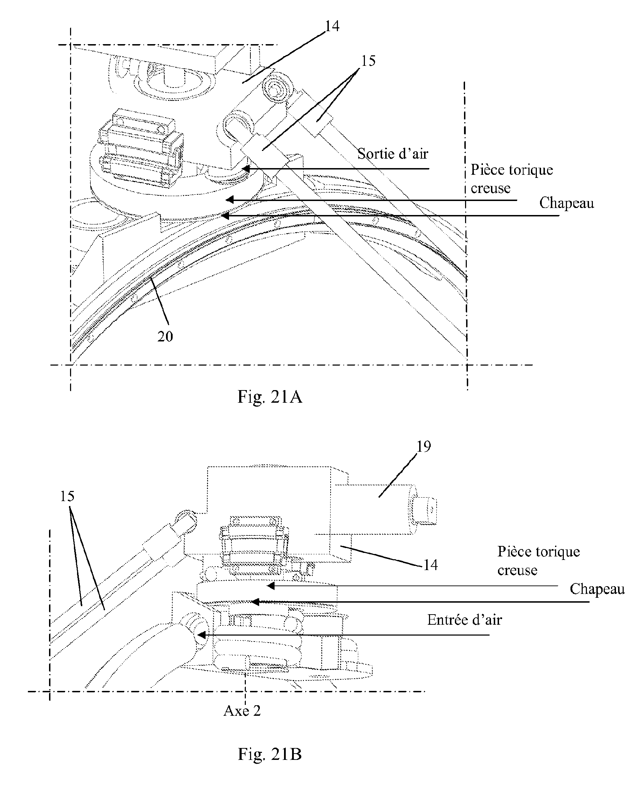

Des exemples de solutions, pour gérer le cheminement des flexibles au niveau des deuxième et troisième articulations 8' et 8" (axe 2 et axe 3), sont représentés sur les figures 21A à 21F. Dans les deux cas, les fonctions d'alimentation en courant et de circulation d'air sont découplées.

Au niveau de l'articulation 8' (axe 2), un joint tournant de forme torique est créé autour de la liaison pivot que constitue l'articulation 8' (axe 2 / figures 21A et 21B). Ce joint est constitué d'une pièce torique creuse et d'un chapeau plaqué contre cette dernière pour assurer l'étanchéité. L'air circule dans la cavité de la pièce torique pour passer entre les deux ensembles en mouvement relatif au niveau de l'articulation 8'.

Pour cette articulation 8', le câble d'alimentation ne passe pas par l'axe de rotation de la liaison.

Afin de gérer la variation de longueur du câble qui en résulte, un système d'enroulement est mis en place. Ce système est composé d'une surface cylindrique située sur l'arbre de rotation 17 fournissant l'axe 2 (figure 21C et 21D), autour de laquelle s'enroule le flexible, et d'une poulie rappelée en rotation sur laquelle chemine ensuite le câble. Le câble pénètre à l'intérieur de cette poulie pour en sortir au niveau de son axe de rotation. Dès qu'une rotation autour de l'articulation provoque un allongement du brin de câble, l'excédent est enroulé sur la poulie automatiquement.

Au niveau de l'articulation 8" (axe 3), la variation de longueur d'arc (figure 9) est gérée, pour l'aspiration d'air, par l'utilisation d'un flexible compressible. La variation de longueur est donc absorbée en réduisant la longueur apparente du flexible. La variation de longueur du câble d'alimentation est prise en compte par l'installation d'une poulie avec rappel par ressort de torsion.

La mise en oeuvre de l'installation robotisée 1 selon l'invention, dans l'application SMT ou une application analogue, suppose un positionnement initial du patient 3' afin de recaler le centre PF du mécanisme principal sphérique (premier sous-ensemble 7) avec le centre de la tête 3. Le siège 6 est donc muni de deux dispositifs de réglage 32 et 32', selon des directions horizontale et verticale, associés chacun à un organe de commande manuelle 32" respectif (par exemple une manivelle). La procédure de positionnement comprendra un réglage manuel facilité par ce dispositif de réglage continu selon deux degrés de liberté, et sera basée soit sur l'estimation de la position de la tête à partir d'un localisateur externe, soit par un indication optique du centre PF du mécanisme sphérique (sous-ensemble 7) projetée sur le patient 3'.

Deux stratégies opératoires peuvent être envisagées après un calage initial de la tête du patient dans le volume 9 par rapport au point focal PF des premier et second sous-ensembles 7 et 7' (projection d'une croix sur le front du patient).

Selon une première possibilité, le patient et en particulier la tête du patient est maintenu en position après calage, de manière à éviter un décalage entre la trajectoire prédéterminée de la sonde 2 et les zones à traiter.

Selon une seconde possibilité, autorisant une certaine liberté de mouvements du patient, le patient est muni de marqueurs passifs (par exemple un bandeau à marqueurs enserrant sa tête ou un masque posé sur le visage) pouvant être détectés par des capteurs de position correspondant (par exemple deux caméras infrarouges ou optiques à 90°). Les informations de ces capteurs sont fournies de manière continue à l'unité de commande et de pilotage de l'installation robotisée 1, qui modifiera la trajectoire précalculée de la sonde 2 en fonction des possibles mouvements du patient, à la manière d'un asservissement en mouvement temps réel. Un tel système de localisation est de suivi est, par exemple, connu par la société NDI Inc. sous la désignation POLARIS.

Les différents actionneurs des articulations rotatoires des trois sous-ensembles 7, 7' et 7" consistent préférentiellement en des moteurs électriques (par exemple à courant continu ou à excitation harmonique) associés à des mécanismes de transmission et/ou de réduction du mouvement et à des capteurs de positions et/ou codeurs, bien connus dans le domaine de la robotique, l'ensemble de ces actionneurs étant piloté par une unité de commande et de pilotage adaptée, par exemple une unité informatique, calculant également et stockant la trajectoire de **la sonde** 2, ce à partir de données programmées ou déduites d'images antérieures.

L'installation robotisée 1 selon l'invention permet ainsi de satisfaire les exigences précitées de précision (positionnement à 1 mm près) et de sécurité (active et passive), et de répondre aux contraintes associées à une procédure automatisée en milieu médical.

La présente invention a également pour objet un appareil de traitement par stimulation magnétique transcrânienne, comprenant essentiellement une sonde 2 comprenant une bobine de stimulation magnétique et portée par une installation robotisée 1.

Cet appareil est caractérisé en ce que l'installation robotisée 1 consiste en une installation telle que décrite précédemment et illustrée à titre d'exemple aux figures annexées, et qui intègre un dispositif robotique 5 à sept degrés de liberté, lequel est apte à réaliser un positionnement et un déplacement automatiques de ladite sonde autour de la tête d'un patient en fonction d'une trajectoire déterminée préalablement, ce sous le contrôle d'une unité de commande et de pilotage.

La fourniture d'un dispositif robotique 5 avec sept degrés de liberté, c'est-à-dire un degré de liberté redondant, permet un comportement cinématique satisfaisant et la garantie d'un déplacement et d'un positionnement précis au niveau de chaque articulation, ainsi qu'une sécurité optimisée au niveau contrôle.

Préférentiellement cet appareil comprend également un système de localisation, préférentiellement en position et en orientation, de la tête 3 du patient 3' dans la volume 9 recevant le patient, coopérant avec l'unité de commande et de pilotage en vue de réaliser un calage de la tête 3 par rapport au point focal PF du premier sous-ensemble 7 du dispositif robotique 5 et un asservissement, en fonction des mouvements de la tête 3, de la position de la sonde 2 par l'intermédiaire de l'installation robotisée 1, sous le contrôle de l'unité de commande et de pilotage et en fonction des signaux ou données fourni(e)s par le système de localisation.

Un tel appareil permet de remplacer l'opérateur durant une procédure de SMT, en garantissant la précision et la sécurité requises.

Les caractéristiques principales d'un appareil de stimulation magnétique transcrânienne du type précité ressortent notamment du document "Robotic image-guided transcranial magnetic stimulation", Lebossé C. et al, Computer assisted radiology and surgery, volume 1, Osaka, Japon 2006, dont la totalité du contenu est intégrée à la présente par référence.

Enfin l'invention est utilisable dans le cadre d'un procédé de stimulation magnétique transcrânienne mettant en oeuvre l'appareil décrit ci-dessus.

Ce procédé consiste essentiellement à réaliser successivement les étapes suivantes :
- réalisation de plusieurs images du cerveau et de la tête du patient à traiter, par exemple par IRM ou IRMf ;
- construction d'un modèle tridimensionnel du cerveau et de la tête du patient ;
- détermination des régions corticales à stimuler et de leur ordre respectif dans la séquence de stimulation, ainsi que de la fréquence, de la durée et, le cas échéant, de la puissance de stimulation pour chaque région ;
- calcul par l'unité de commande et de pilotage de l'appareil, ou par une unité de calcul externe de la trajectoire du centre fonctionnel de la bobine de la sonde autour de la tête du patient pour stimuler de façon optimale chaque région corticale concernée en fonction des spécificités prédéterminées ;
- positionnement du patient dans l'installation robotisée et réalisation du calage ou cadrage de sa tête par rapport au point focal de l'installation robotisée et au modèle tridimensionnel ;
- lancement et exécution autonome de la procédure de stimulation avec déplacement contrôlé de la sonde par le dispositif robotique, avec éventuellement acquisition de la localisation et de l'orientation de la sonde pour une analyse postérieure au traitement.

Plus précisément, après identification des raisons corticales à stimuler, la trajectoire du centre de la bobine de la sonde 2 est calculée, ainsi que l'orientation de ladite sonde pendant la trajectoire, pour une stimulation optimale desdites régions, ce sur la base d'une reconstitution tridimensionnelle de la tête du patient.

La vitesse de déplacement de la sonde, ainsi que la puissance, la fréquence et le nombre de séquences de stimulation par région à stimuler sont calculés ou programmés.

Le calcul de la trajectoire requise, qui prend également en compte les contraintes physiques et mécaniques du dispositif robotique (limites angulaires de rotation des articulation, limites en terme de vitesse de rotation des articulations, évitement de collision), peut par exemple être réalisé au niveau de l'unité de commande et de pilotage par l'intermédiaire d'un algorithme de planification de mouvement probabilistique, par exemple basé sur un algorithme utilisant des échantillons et dérivé de "Probabilistic road maps for path planning in high dimensional configuration spaces", Kavraki L. et al., IEE Transaction on robotics and automation, 1996, volume 12, pages 566 à 580.

Par ailleurs, une technique de contrôle de vélocité pseudo-inverse est utilisée pour réaliser le déplacement de la sonde 2 (voir par exemple : "Advanced robotics: Redundancy and Optimization", Nakamura Y., Addison-Wesley Longman Publishing, Boston, 1991).

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention tel que défini dans les revendications.

## Revendications

1. Installation robotisée **(1)** pour le positionnement et le déplacement guidés et contrôlés d'une sonde de stimulation magnétique transcrânienne, au niveau ou autour de la tête **(3)** d'un patient **(3'),**
ladite installation **(1)** comprenant essentiellement une structure support **(4)** sur laquelle sont montés les éléments constitutifs d'un dispositif robotique **(5)** formant une chaîne cinématique série et portant à son extrémité libre et contrôlée en position **la sonde (2)** précité, un dispositif **(6)** réglable de support et de maintien du patient **(3'),** sensiblement en position assise, faisant partie de ou étant associé à ladite structure support **(4),**
ledit dispositif robotique (5) **comprenant deux** (7, 7') sous-ensembles cinématiques mutuellement associés en série, **à savoir,** d'une part, un premier sous-ensemble (7) sous la forme d'un mécanisme à articulations rotatoires (8, 8', 8"), solidaire de la structure support (4) par une première articulation (8) et correspondant à un arrangement cinématique sphérique de type série à trois degrés de liberté, les éléments d'articulation (8, 8', 8") étant tous situés en dehors du volume (9) susceptible de recevoir le patient (3') et leurs axes de rotation (axe 1, axe 2 et axe 3) étant concourants en un point focal (PF) correspondant sensiblement au centre hypothétique de la tête (3) du patient (3') en position d'intervention, **et,** d'autre part, un deuxième sous-ensemble (7') sous la forme d'un mécanisme à translation linéaire selon un axe (axe 4) passant par le point focal (PF) précité et solidaire de la partie mobile (10) de la troisième articulation (8") en série du premier sous-ensemble (7),
**installation (1) caractérisée en ce que le dispositif robotique (5) comprend en outre** un troisième sous ensemble (7") sous la forme d'un second mécanisme à **trois** articulations rotatoires **en série (11, 11', 11") formant un poignet sphérique à trois degrés de liberté, ce troisième sous-ensemble (7") étant** solidaire de la partie mobile (12) du second sous-ensemble (7') et **le dispositif robotique (5) étant ainsi redondant à sept degrés de liberté.**

2. Installation selon la revendication 1, **caractérisée en ce que** la première articulation rotatoire (8) du premier sous-ensemble (7) consiste en une liaison à glissière circulaire, comprenant un rail de guidage (13), préférentiellement un rail double, en forme d'arc de cercle, sensiblement semi-circulaire, solidaire de la structure support (4), et un chariot mobile (14) apte à circuler sur ledit rail (13), et dont le déplacement est commandé par une tringlerie de transmission (15) reliée à un actionneur (16), le mouvement circulaire s'effectuant dans le plan médian du patient (3) en position d'intervention et ledit rail de guidage (13) présentant un positionnement et une extension tels qu'il s'étend autour de la tête (3) dudit patient (3') sensiblement de la base arrière du crâne jusqu'au front.

3. Installation selon la revendication 2, **caractérisée en ce que** la seconde articulation rotatoire (8') du premier sous-ensemble (7) consiste en une articulation axiale avec un arbre (17) monté à rotation dans un palier (18) ménagé dans le chariot mobile (14) faisant partie de la première articulation rotatoire (8), le déplacement en rotation dudit arbre étant commandé par un actionneur (19), par exemple une unité moteur-réducteur, porté par ledit chariot (14).

4. Installation selon l'une quelconque des revendications 1 à 3 **caractérisée en ce que** la troisième articulation rotatoire (8") du premier sous-ensemble (7) consiste en une liaison à glissière circulaire sous la forme de deux rails (20, 21) coopérant de manière coulissante et réalisé chacun en forme d'arc de cercle, lesdits rails (20 et 21) pouvant être déplacés relativement l'un par rapport à l'autre entre une disposition repliée dans laquelle ils sont sensiblement superposés ou chevauchant sur toute leur longueur, et une disposition déployée, dans laquelle ils ne sont plus superposés que sur une faible partie.

5. Installation selon les revendications 3 et 4, **caractérisée en ce que** l'un (20) des rails (20, 21) formant la troisième articulation rotatoire (8") est assemblé rigidement avec l'arbre (17) de la seconde articulation rotatoire (8') les deux rails (20 et 21) pouvant être bloqués en position l'un par rapport à l'autre constructivement, par liaison mécanique ou par blocage de l'actionneur assurant leur mouvement relatif mutuel.

6. Installation selon l'une quelconque des revendications 2, 3 et 5, **caractérisée en ce que** le chariot mobile (14) de la première articulation rotatoire (8) est de manière permanente sollicité en position extrême supérieure par un mécanisme de rappel à tension constante (22), par exemple à câble (22').

7. Installation selon l'une quelconque des revendications 3 et 5, **caractérisée en ce qu'**un dispositif (23) de blocage en rotation de l'arbre (17) est associé à l'actionneur (19), par exemple sous la forme d'un frein actif en l'absence d'alimentation électrique au niveau de ce dernier, par exemple du type électromagnétique, ledit dispositif (23) pouvant être débloqué par un opérateur par l'intermédiaire d'une commande manuelle d'alimentation en courant, afin d'autoriser une libre rotation de l'arbre (17) autour de son axe de rotation (axe 2).

8. Installation selon l'une quelconque des revendications 4 et 5, **caractérisée en ce que** l'actionneur de la troisième articulation rotatoire (8") du premier sous-ensemble (7) réalise un blocage en position mutuel des deux rails (20 et 21) en l'absence d'alimentation électrique.

9. Installation selon l'une quelconque des revendications 1 à 8, **caractérisée en ce qu'**un capteur d'effort, notamment de pression, est associé au deuxième sous-ensemble (7'), permettant de réaliser un asservissement en effort dudit deuxième sous-ensemble (7') dans la direction de translation (axe 4) sensiblement perpendiculaire à la surface de la tête (3) du patient (3') en position d'intervention, ce sous-ensemble (7') comprenant en outre un mécanisme de rappel, par exemple de type mécanique, sollicitant **la sonde** (2) à distance de la surface de la tête (3) du patient (3').

10. Installation selon la revendication 1, **caractérisée en ce que** les axes de rotation (axe 5, axe 6, axe 7) des articulations rotatoires (11, 11', 11") **du troisième sous-ensemble (7")** sont concourants.

11. Installation selon l'une quelconque des revendications **1 à 10, caractérisée en ce que** le troisième sous-ensemble (7") formant poignet sphérique est essentiellement constitué par une première articulation (11) formée d'un rail (24) en arc de cercle solidaire de la partie mobile (12) du deuxième sous-ensemble (7') et sur lequel circule un chariot (25) dont le déplacement est commandé par un actionneur (26), par une seconde articulation (11') formée de deux bras (27) montés rigidement sur le chariot mobile (25) par l'une de leur extrémités et portant à leurs extrémités opposées chacun un chariot fixe (28) coopérant chacun avec une portion de rail mobile (29) en forme d'arc de cercle et parallèles entre elles, et par une troisième articulation (11") sous la forme d'une platine à palier (30), solidaire des rails mobiles (29) et apte à recevoir avec faculté de rotation (axe 7) un moyeu (31) solidaire de **la** sonde (2).

12. Installation selon l'une quelconque des revendications 1 à 8, **10 et 11, caractérisée en ce qu'**un capteur d'effort, en particulier un capteur de pression, est intégré dans **la sonde** (2) porté par un dispositif robotique (5), ledit capteur d'effort faisant partie d'un asservissement en effort du deuxième sous-ensemble (7').

13. Appareil de traitement par stimulation magnétique transcrânienne, comprenant essentiellement une sonde comprenant une bobine de stimulation magnétique et portée par une installation robotisée, appareil **caractérisé en ce que** l'installation robotisée (1) consiste en une installation selon l'une quelconque des revendications 1 à **12** intégrant un dispositif robotique (5) à sept degrés de liberté, lequel est apte à réaliser un positionnement et un déplacement automatiques de ladite sonde (2) autour de la tête (3) d'un patient (3') en fonction d'une trajectoire déterminée préalablement, ce sous le contrôle d'une unité de commande et de pilotage.

14. Appareil selon la revendication **13, caractérisé en ce qu'**il comprend également un système de localisation, préférentiellement en position et en orientation, de la tête (3) du patient (3') dans la volume (9) recevant le patient, coopérant avec l'unité de commande et de pilotage en vue de réaliser un calage de la tête (3) par rapport au point focal (PF) du premier sous-ensemble (7) du dispositif robotique (5) et un asservissement, en fonction des mouvements de la tête (3), de la position de la sonde (2) par l'intermédiaire de l'installation robotisée (1), sous le contrôle de l'unité de commande et de pilotage et en fonction des signaux ou données fourni(e)s par le système de localisation.

## Patentansprüche

1. Robotergesteuerte Einrichtung (1) zur gelenkten und kontrollierten Positionierung und Bewegung einer Sonde zur transkraniellen Magnetstimulation im Bereich des oder um den Kopf (3) eines Patienten (3'),
wobei die genannte Einrichtung (1), im Wesentlichen eine Tragstruktur (4) umfassend, auf der die Komponenten einer Robotervorrichtung (5) angebracht sind, die eine offene kinematische Kette bilden, an ihrem freien Ende die genannte Sonde (2) in kontrollierter Stellung trägt, wobei eine einstellbare Vorrichtung (6) zum Tragen und Halten des Patienten (3') im Wesentlichen in Sitzstellung Teil der genannten Tragstruktur oder mit ihr verbunden ist,
wobei die genannte Robotervorrichtung (5) zwei kinematische Untereinheiten (7, 7') aufweist, die miteinander in Reihe verbunden sind, nämlich einerseits eine erste Untereinheit (7) in Form eines Mechanismus mit Drehgelenken (8, 8', 8"), die mit der Tragstruktur (4) über ein erstes Gelenk (8) verbunden ist und einer kugelförmigen, offenen kinematischen Anordnung mit drei Freiheitsgraden entspricht, wobei sich alle Gelenkelemente (8, 8', 8") außerhalb des Volumens (9) befinden, das gegebenenfalls den Patienten (3') empfängt, und ihre Drehachsen (Axe 1, Axe 2 und Axe 3) in einem Brennpunkt (PF) konvergieren, der im Wesentlichen dem hypothetischen Zentrum des Kopfes (3) des Patienten (3') in Behandlungsstellung entspricht, und andererseits eine zweite Untereinheit (7') in Form eines Mechanismus zur linearen Verschiebung längs einer Achse (Axe 4), die durch den genannten Brennpunkt (PF) verläuft und mit dem beweglichen Teil (10) des dritten Gelenks (8") in Reihe der ersten Untereinheit (7) verbunden ist,
Einrichtung (1), **dadurch gekennzeichnet, dass** die Robotervorrichtung (5) außerdem eine dritte Untereinheit (7") in Form eines zweiten Mechanismus mit drei Drehgelenken in Reihe (11, 11', 11") umfasst, die ein kugelförmiges Handgelenk mit drei Freiheitsgraden bildet, wobei diese dritte Untereinheit (7") am beweglichen Teil (12) der zweiten Untereinheit (7') befestigt ist, womit also die Robotervorrichtung (5) mit sieben Freiheitsgraden redundant ist.

2. Einrichtung nach Patentanspruch 1, **dadurch gekennzeichnet, dass** das erste Drehgelenk (8) der ersten Untereinheit (7) aus einer Verbindung mit kreisförmiger Gleitschiene besteht, eine Führungsschiene (13), vorzugsweise eine Zwillingsschiene in Kreisbogenform, im Wesentlichen halbkreisförmig, umfassend, die an der Tragstruktur (4) befestigt ist, und einen beweglichen Wagen (14), der in der Lage ist, auf der genannten Schiene (13) zu laufen, und dessen Bewegung durch ein Übertragungsgestänge (15) veranlasst wird, das mit einem Wirkglied (16) verbunden ist, wobei die Kreisbewegung in der Mittelebene des Patienten (3) in Behandlungsstellung erfolgt und die genannte Führungsschiene (13) eine Stellung und Ausdehnung hat, derart dass sie sich um den Kopf (3) des genannten Patienten (3') im Wesentlichen von der hinteren Schädelbasis bis zur Stirn erstreckt.

3. Einrichtung nach Patentanspruch 2, **dadurch gekennzeichnet, dass** das zweite Drehgelenk (8') der ersten Untereinheit (7) aus einem Achsengelenk mit einer Welle (17) besteht, die drehbar in einem Lager (18) montiert ist, das im beweglichen Wagen (14) ausgebildet ist, der Teil des ersten Drehgelenks (8) ist, wobei die Drehung der genannten Welle durch ein Wirkglied (19) veranlasst wird, beispielsweise eine Einheit aus Motor und Getriebe, das vom genannten Wagen (14) getragen wird.

4. Einrichtung nach irgendeinem der Patentansprüche 1 bis 3, **dadurch gekennzeichnet, dass** das dritte Drehgelenk (8") der ersten Untereinheit (7) aus einer Verbindung mit kreisförmiger Gleitschiene in Form von zwei Schienen (20, 21), die gleitend zusammenwirken und jeweils kreisbogenförmig ausgebildet sind, besteht, wobei die genannten Schienen (20 und 21) relativ zueinander zwischen einer eingezogenen Anordnung, in der sie im Wesentlichen übereinander liegen oder sich auf ihrer ganzen Länge überlappen, und einer ausgezogenen Stellung, in der sie nur noch in einem geringen Teil übereinander liegen, verschoben werden können.

5. Einrichtung nach den Patentansprüchen 3 und 4, **dadurch gekennzeichnet, dass** eine (20) der Schienen (20, 21), die das dritte Drehgelenk (8") bilden, starr mit der Welle (17) des zweiten Drehgelenks (8') verbunden ist, wobei die beiden Schienen (20 und 21) in ihrer Stellung relativ zueinander konstruktiv, durch mechanische Verbindung oder durch Blockierung des Wirkgliedes, das ihre Relativbewegung zueinander sicherstellt, blockiert werden können.

6. Einrichtung nach irgendeinem der Patentansprüche 2, 3 und 5, **dadurch gekennzeichnet, dass** der bewegliche Wagen (14) des ersten Drehgelenks (8) durch einen Rückstellmechanismus konstanter Spannung (22), beispielsweise mit einem Drahtseil (22'), permanent in die äußerste obere Stellung vorgespannt wird.

7. Einrichtung nach irgendeinem der Patentansprüche 3 und 5, **dadurch gekennzeichnet, dass** eine Vorrichtung (23) zur Blockierung der Drehung der Welle (17) mit dem Wirkglied (19) verbunden ist, beispielsweise in Form einer Bremse, beispielsweise vom elektromagnetischen Typ, die bei Abwesenheit der Versorgung mit elektrischem Strom in dessen Bereich tätig ist, wobei die genannte Vorrichtung (23) von einem Bediener mit Hilfe einer manuellen Betätigung der Stromversorgung freigesetzt werden kann, um eine freie Drehung der Welle (17) um ihre Drehachse (Axe 2) zu erlauben.

8. Einrichtung nach irgendeinem der Patentansprüche 4 und 5, **dadurch gekennzeichnet, dass** das Wirkglied des dritten Drehgelenks (8") der ersten Untereinheit (7) eine Blockierung der beiden Schienen (20 und 21) in ihrer Stellung zueinander bei Abwesenheit der Versorgung mit elektrischem Strom vornimmt.

9. Einrichtung nach irgendeinem der Patentansprüche 1 bis 8, **dadurch gekennzeichnet, dass** ein Belastungssensor, insbesondere ein Drucksensor, mit der zweiten Untereinheit (7') verbunden ist, der eine Regulierung der Kraft der genannten zweiten Untereinheit (7') in der Verschiebungsrichtung (Axe 4), im Wesentlichen senkrecht zum Kopf (3) des Patienten (3') in der Behandlungsstellung, erlaubt, wobei diese Untereinheit (7') außerdem einen Rückstellmechanismus beispielsweise mechanischer Art umfasst, der die Sonde (2) in einen Abstand von der Oberfläche des Kopfes (3) des Patienten (3') vorspannt.

10. Einrichtung nach Patentanspruch 1, **dadurch gekennzeichnet, dass** die Drehachsen (Axe 5, Axe 6, Axe 7) der Drehgelenke (11, 11', 11 ") der dritten Untereinheit (7") konvergieren.

11. Einrichtung nach irgendeinem der Patentansprüche 1 bis 10, **dadurch gekennzeichnet, dass** die dritte Untereinheit (7"), die ein kugelförmiges Handgelenk bildet, im Wesentlichen aus einem ersten Gelenk (11) besteht, das aus einer kreisbogenförmigen Schiene (24) besteht, die am beweglichen Teil (12) der zweiten Untereinheit (7') befestigt ist und auf der ein Wagen (25) läuft, dessen Verschiebung von einem Wirkglied (26) veranlasst wird, aus einem zweiten Gelenk (11'), das aus zwei Armen (27) besteht, die mit einem ihrer Enden starr am beweglichen Wagen (25) angebracht sind und an ihrem entgegengesetzten Ende jeweils einen feststehenden Wagen (28) tragen, der jeweils mit einem Teil der kreisbogenförmigen beweglichen Schiene (29) zusammenwirkt, und die zueinander parallel sind, und aus einem dritten Gelenk (11") in Form einer Halteplatte mit Lager (30), die an den beweglichen Schienen (29) befestigt und in der Lage ist, drehbar (Axe 7) eine Nabe (31) aufzunehmen, die an der Sonde (2) befestigt ist.

12. Einrichtung nach irgendeinem der Patentansprüche 1 bis 8, 10 und 11, **dadurch gekennzeichnet, dass** ein Belastungssensor, insbesondere ein Drucksensor, in die Sonde (2) eingebaut ist, die von einer Robotervorrichtung (5) getragen wird, wobei der genannte Belastungssensor Teil einer Reguliervorrichtung der Kraft der zweiten Untereinheit (7') ist.

13. Gerät zur Behandlung durch transkranielle Magnetstimulation, im Wesentlichen eine Sonde umfassend, die eine Spule zur Magnetstimulation umfasst und von einer robotergesteuerten Einrichtung getragen wird, Gerät, **dadurch gekennzeichnet, dass** die robotergesteuerte Einrichtung (1) aus einer Einrichtung nach irgendeinem der Patentansprüche 1 bis 12 besteht, die eine Robotervorrichtung (5) mit sieben Freiheitsgraden umfasst, die unter der Kontrolle einer Steuer- und Lenkeinheit in der Lage ist, eine automatische Positionierung und Bewegung der genannten Sonde (2) um den Kopf (3) eines Patienten (3') auf einer vorher festgelegten Bahn vorzunehmen.

14. Gerät nach Patentanspruch 13, **dadurch gekennzeichnet, dass** es auch ein System zur Lokalisierung, vorzugsweise der Stelle und der Orientierung, des Kopfes (3) des Patienten (3') im Volumen (9), das den Patienten empfängt, umfasst, das mit der Steuer- und Lenkeinheit zusammenarbeitet, um eine Feststellung des Kopfes (3) relativ zum Brennpunkt (PF) der ersten Untereinheit (7) der Robotervorrichtung (5) vorzunehmen und in Abhängigkeit von den Bewegungen des Kopfes (3) eine Regelung der Stellung der Sonde (2) durch eine robotergesteuerte Einrichtung (1) unter Kontrolle der Steuer- und Lenkeinheit und in Abhängigkeit von den Signalen oder Daten, die das Lokalisierungssystem ausgibt.

## Claims

1. Robotised installation (1) for the guided and controlled positioning and movement of a transcranial magnetic stimulation probe, at or around the head (3) of a patient (3'), wherein said installation (1) essentially comprises a support structure (4) on which are mounted the constituent elements of a robotic device (5) forming a serial kinematic chain and carrying the aforementioned probe (2) at its free and position-controlled end, an adjustable device (6) for supporting and holding the patient (3'), essentially in the seated position, forming part of or being connected to said support structure (4),
said robotic device (5) comprising two (7, 7') kinematic sub-assemblies that are mutually connected in series, namely on the one hand a first sub-assembly (7) in the form of a rotary articulation mechanism (8, 8', 8"), integral with the support structure (4) by a first articulation (8) and corresponding to a serial-type, spherical kinematic arrangement with three degrees of freedom, whereby the articulation elements (8, 8', 8") are all located outside of the space (9) that can accommodate the patient (3') and whereby their axes of rotation (axis 1, axis 2, and axis 3) are concurrent at a focal point (PF) that corresponds approximately to the hypothetical centre of the head (3) of the patient (3') in an intervention position, and, on the other hand, a second sub-assembly (7') in the form of a mechanism with linear translation along an axis (axis 4) that passes through the aforementioned focal point (PF) and that is integral with the moving part (10) of the third articulation (8") in series of the first sub-assembly (7),
the installation (1) **characterised in that** the robotic device (5) also comprises a third sub-assembly (7") in the form of a second rotary articulation mechanism in series (11, 11', 11") forming a spherical cuff with three degrees of freedom, said third sub-assembly (7") being connected to the mobile part (12) of the second subassembly (7') and the robotic device (5) being thus redundant at seven degrees of freedom.

2. Installation according to claim 1, **characterised in that** the first rotary articulation (8) of the first sub-assembly (7) consists in a circular sliding connection, comprising a guide rail (13), preferably a double rail, in the form of an essentially semi-circular arc, integral with the support structure (4), and a moving carriage (14) that can circulate on said rail (13) and whose movement is controlled by a transmission linkage (15) that is connected to an actuator (16), whereby the circular movement is carried out in the median plane of the patient (3) in intervention position, and said guide rail (13) has a positioning and an extension such that it extends around the head (3) of said patient (3') essentially at the rear base of the skull up to the forehead.

3. Installation according to claim 2, **characterised in that** the second rotary articulation (8') of the first sub-assembly (7) consists of an axial articulation with a shaft (17) that is mounted to rotate in a bearing (18) that is provided in the moving carriage (14) that is part of the first rotary articulation (8), whereby the movement in rotation of said shaft is controlled by an actuator (19), for example a geared motor unit, supported by said carriage (14).

4. Installation according to any one of claims 1 to 3, **characterised in that** the third rotary articulation (8") of the first sub-assembly (7) consists of a circular slide connection in the form of two rails (20, 21) that work in a sliding manner and that are each made in the shape of an arc, whereby said rails (20 and 21) can be moved relative to one another between a folded arrangement in which they are essentially placed on top of one another, or overlap over their entire length, and a deployed arrangement, in which they are no longer placed on top of one another except on a small part.

5. Installation according to claims 3 and 4, **characterised in that** one (20) of the rails (20, 21) that forms the third rotary articulation (8") is assembled rigidly with the shaft (17) of the second rotary articulation (8') of the two rails (20 and 21) that can be structurally locked in position relative to one another, by mechanical connection or by locking of the actuator, ensuring their mutual relative movement.

6. Installation according to any one of claims 2, 3 and 5, **characterised in that** the moving carriage (14) of the first rotary articulation (8) is permanently stressed in the upper end position by a constant tension return mechanism (22), for example with a cable (22').

7. Installation according to any one of claims 3 and 5, **characterised in that** a device (23) for locking the shaft (17) in rotation is connected to the actuator (19), for example in the form of an active brake in the event of a power failure at the level of the latter, for example of the electromagnetic type, whereby said device (23) can be unlocked by an operator by means of a manual control for current supply, so as to allow a free rotation of the shaft (17) around its axis of rotation (axis 2).

8. Installation according to any one of claims 4 and 5, **characterised in that** the actuator of the third rotary articulation (8") of the first sub-assembly (7) carries out a locking in mutual position of the two rails (20 and 21) in the event of a power failure.

9. Installation according to any one of claims 1 to 8, **characterised in that** a stress sensor, in particular a pressure sensor, is coupled to the second sub-assembly (7'), making it possible to produce a force control of said second sub-assembly (7') in the translation direction (axis 4) that is essentially perpendicular to the surface of the head (3) of the patient (3') in intervention position, whereby this sub-assembly (7') also comprises a return mechanism, for example of the mechanical type, forcing the probe (2) remotely from the surface of the head (3) of the patient (3').

10. Installation according to claim 1, **characterised in that** the axes of rotation (axis 5, axis 6, axis 7) of said rotary articulations (11, 11', 11") of the third sub-assembly (7") are concurrent.

11. Installation according to any one of claims 1 to 10, **characterised in that** the third sub-assembly (7") that forms the spherical cuff essentially consists of a first articulation (11) that is formed by a rail (24) in an arc that is integral with the moving part (12) of the second sub-assembly (7') and on which a carriage (25) circulates whose movement is controlled by an actuator (26), by a second articulation (11') that is formed by two arms (27) that are mounted rigidly on the moving carriage (25) by one of their ends and each carrying at their opposite ends a stationary carriage (28) that each works with a moving rail portion (29) in the shape of an arc, and parallel between them, and by a third articulation (11") in the form of a bearing plate (30), integral with the moving rails (29) and able to accommodate with the ability to rotate (axis 7) a hub (31) that is integral with probe (2).

12. Installation according to any one of claims 1 to 8, 10 and 11, **characterised in that** a stress sensor, in particular a pressure sensor, is integrated into the probe (2) that is carried by a robotic device (5), whereby said stress sensor is part of a force control of the second sub-assembly (7').

13. Device for treatment by transcranial magnetic stimulation, essentially comprising a probe that comprises a magnetic stimulation coil and that is supported by a robotised installation, a device that is **characterised in that** the robotised installation (1) consists of an installation according to any one of claims 1 to 12, integrating a robotic device (5) with seven degrees of freedom, which is able to produce an automatic positioning and movement of said probe (2) around the head (3) of a patient (3') based on a previously determined path, this under the monitoring of a control and guiding unit.

14. Device according to claim 13, **characterised in that** it also comprises a locating system, preferably in position and in orientation, of the head (3) of the patient (3') in the space (9) that accommodates the patient, working with the control and guiding unit for the purpose of carrying out an adjustment of the head (3) relative to the focal point (PF) of the first sub-assembly (7) of the robotic device (5) and a control, based on movements of the head (3), of the position of the probe (2) by means of the robotised installation (1), under the monitoring of the control and guiding unit and based on signals or data provided by the locating system.
